(19) Europäisches Patentamt
European Patent Office
Office européen des brevets

(11) Veröffentlichungsnummer: **0 178 414**
**B1**

(12) # EUROPÄISCHE PATENTSCHRIFT

(45) Veröffentlichungstag der Patentschrift: **13.01.88**

(51) Int. Cl.⁴: **C 08 G 14/06**, C 08 G 59/40

(21) Anmeldenummer: **85110081.8**

(22) Anmeldetag: **12.08.85**

(54) **Chemisch härtbare Harze aus 1-Oxa-3-aza-tetralin-gruppen enthaltenden Verbindungen und cycloaliphatischen Epoxid-harzen, Verfahren zu deren Herstellung und Härtung sowie die Verwendung solcher Harze.**

(30) Priorität: **14.09.84 DE 3433851**

(43) Veröffentlichungstag der Anmeldung: **23.04.86 Patentblatt 86/17**

(45) Bekanntmachung des Hinweises auf die Patenterteilung: **13.01.88 Patentblatt 88/2**

(84) Benannte Vertragsstaaten: **AT BE CH DE FR GB IT LI LU NL SE**

(56) Entgegenhaltungen: **GB-A-1 437 814**

(73) Patentinhaber: **Gurit- Essex AG, CH- 8807 Freienbach (CH)**

(72) Erfinder: **Schreiber, Herbert, Seeblick 3, CH- 8832 Wollerau (CH)**

(74) Vertreter: **Rottmann, Maximilian R., c/o Rottmann + Quehl AG Glattalstrasse 37, CH- 8052 Zürich (CH)**

**EP 0 178 414 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein neues chemisch härtbares Harz aus 1-Oxa-3-aza-tetralin-gruppen enthaltenden Verbindungen und/oder deren Vorpolymeren und cycloaliphatischen Epoxid-harzen mit in einem cycloaliphatischen Ring anellierten Epoxid-gruppen.

1-Oxa-3-aza-tetralin-gruppen enthaltende Verbindungen und deren Vorpolymere sind z. B. aus den Schweizer Patentschriften 574, 978, 579, 113 und 606, 169 bekannt. Sie werden z. B. aus Phenolen durch Umsetzung mit Formaldehyd und einem Amin erhalten etwa gemäss:

(a)

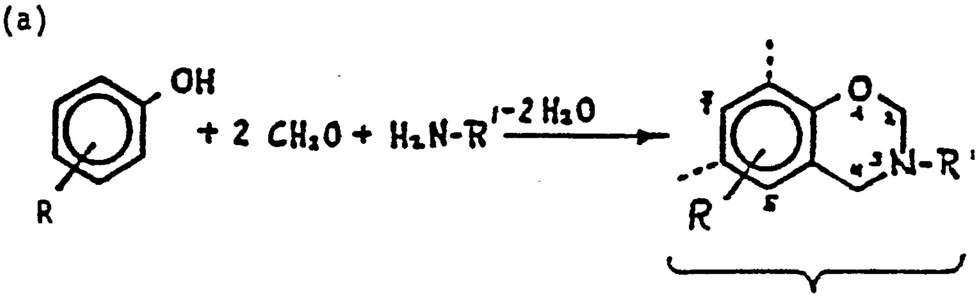

1-Oxa-3-aza-tetralin-gruppe

können aber auch nach andern zu gleichartigen Produkten führenden Verfahren gewonnen werden.

R bedeutet beispielsweise Wasserstoff, Halogen, Alkyl oder Alkoxy und R' einen aliphatischen oder aromatischen Rest.

Im Gegensatz zu anderen, bekannten Kondensationsreaktionen von Phenolen, Aminen und Formaldehyd werden bei dieser Reaktion phenolische OH-Gruppen verbraucht. Aus der analytischen Bestimmung dieser Gruppen im Reaktionsgemisch lässt sich somit gemäss Gleichung (a) die Menge der synthetisierten 1-Oxa-3-aza-tetralin-gruppen ermitteln. Erfindungsgemäss verwendet werden Verbindungen, die mehr als eine 1-Oxa-3-aza-tetralin-gruppe im Molekül aufweisen.

Es ist aus den oben genannten Patentschriften auch bekannt, dass sich diese 1-Oxa-3-aza-tetralin-gruppen enthaltenden Verbindungen mit Epoxid-harzen, eingeschlossen cycloaliphatischen Epoxid-harzen, härten lassen.

Die bisher erhaltenen einschlägigen Produkte sind an sich wertvolle Kunststoffe. Ihre Wärmebeständigkeit ist jedoch beschränkt. Die Martens-Wärmebeständigkeit der bisher erhaltenen gehärteten Harze liegt zwischen 120 bis 135°C mit Spitzenwerten von 160 und 170°C.

Für viele Anwendungsbereiche ist eine weit höhere Wärmebeständigkeit erfotderlich.

Elektrische Isolierstoffe hoher Wärmefestigkeit etwa sind eine konstante Forderung der Elektroindustrie. Glas-, Quarz- oder Kohlenstoff-faset-verstätkte Kunststoffe kommen für zahlreiche neue Anwendungsbereiche in Betracht, wenn sich die Wärmefestigkeit des Kunststoffs wesentlich steigern lässt.

Es wurde nun überraschend gefunden, dass man Kunstharze mit besonders herausragender Wärmebeständigkeit in Kombination mit sehr guten mechanischen Eigenschaften erhält, wenn man Mischungen aus Verbindungen mit mehr als einer 1-Oxa-3-aza-tetralin-gruppe im Molekül und/oder deren Vorpolymere und bestimmten cycloaliphatischen Epoxidharzen mit im cycloaliphatischen Ring anellierten Epoxid-gruppen härtet.

Die Wärmebeständigkeit solcher Kunstharze reicht gewöhnlich über 200°C hinaus. Martenstemperatur und der Glasumwandlungspunkt erreichen Werte von 280°C. Ausserdem zeichnen sich diese Kunstharze durch geringe Sprödigkeit und hohe Biege- und Schlagfestigkeit aus.

Gegenstand der Erfindung sind das chemisch härtbare Harz aus 1-Oxa- 3-aza-tetralin-gruppen enthaltenden Verbindungen und anellierte Epoxid-gruppen enthaltenden cycloaliphatischen Epoxid-harzen sowie Verfahren zu dessen Herstellung und Härtung. Gegenstand der Erfindung ist überdies die Verwendung der Harzmischungen sowie der gehärteten Harze.

Das chemisch härtbare Hatz gemäss vorliegender Erfindung enthält Verbindungen mit mehr als einer 1-Oxa-3-aza-tetralin-gruppe im Molekül und/oder deren Vorpolymere sowie cycloaliphatische Epoxid-harze und ist dadurch gekennzeichnet, dass das Epoxid-harz mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen Ring verknüpft sind.

Verbindungen mit mehr als einer 1-Oxa-3-aza-tetralin-gruppe im Molekül lassen sich aus mehrwertigen Phenolen und/oder Aminen gemäss einem der folgenden Reaktionsschemas (b) oder (c) herstellen oder können nach anderen zu gleichartigen Produkt führenden Verfahren gewonnen werden:

(b)

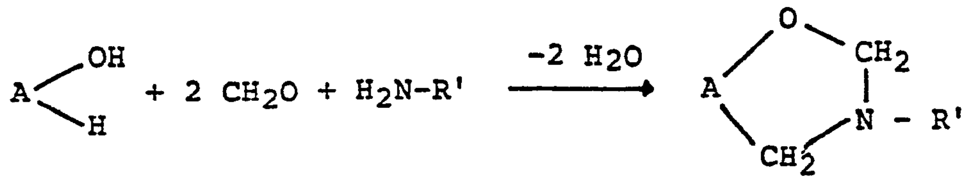


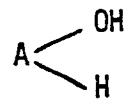

= nter Teil eines Phenols mit n phenolischen OH-Gruppen.

A = Aus 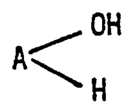

nach Abspaltung von OH und H in ortho-Stellung entstehender Rest.

R' = aliphatischer oder vorzugsweise aromatischer Rest.

n = Zahl grösser als 1, bevorzugt kleiner als 6, insbesondere 1.5 bis 3.

(c)

(c)

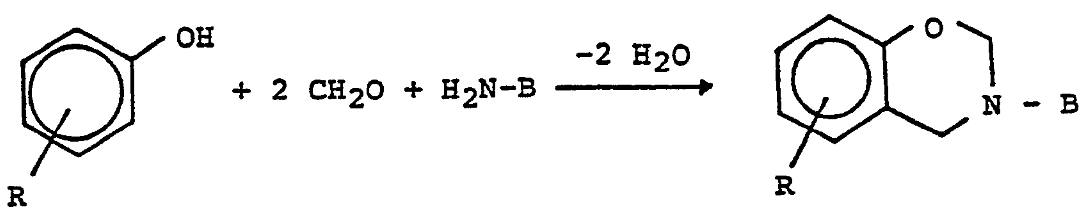


B = mter Teil eines m-wertigen aliphatischen oder bevorzugt aromatischen Restes, der auch Heteroatome, insbesondere Sauerstoff oder Stickstoff, enthalten kann oder mit R substituiert sein kann.

R = Wasserstoff, Halogen, Alkyl oder Alkoxy mit 1 bis 6 C-Atomen.

m = Zahl grösser 1, bevorzugt kleiner als 6, insbesondere 1.5 bis 3.

Die phenolischen Kerne können auch Teile eines kondensierten Ringsystems sein.

Nicht ganzzahlige Werte von n oder m beduten, dass es sich um Mischungen verschieden funktioneller Phenole oder Amine mit den Mittelwerten n oder m handelt.

Für die erfindungsgemässe Anwendung eignen sich als 1-Oxa-3-aza-tetralin-Verbindungen auch solche Reaktionsprodukte, die z. B. gemäss CH-PS 606 169 aus Phenolen, Aminen und Formaldehyd in nicht stöchiometrischen Verhältnissen hergestellt sind. Voraussetzung ist jedoch, dass die Mol-Verhältnisse so gewählt sind, dass sich im Mittel mehr als eine 1-Oxa-3-aza-tetralin-gruppe pro Molekül bilden kann.

Auch Vorpolymere der 1-Oxa-3-aza-tetralin-Verbindungen werden erfindungsgemäss beansprucht. Da die 1-Oxa-3-aza-tetralin-gruppen bei der Polymerisation wegreagieren, können diese Vorpolymeren weniger 1-Oxa-3-aza-tetralin-gruppen enthalten. Entscheidend ist auch hier, dass das intermediär gebildete oder hypothetische monomere Reaktionsprodukt mehr als eine 1-Oxa-3-aza-teralin-gruppe pro Molekül enthält. Dies ist für den Fachmann aus der Funktionalität und den Mengenverhältnisse der Ausgangsstoffe leicht zu berechnen. Eine erfindungsgemässe 1-Oxa-3-aza-tetralin-Verbindung oder deren Vorpolymeres bildet sich z. B. dann, wenn pro Mol mehrfunktionellem Phenol oder Amin mehr als 2 Mol Formaldehyd und mehr als 1 Mol monofunktionelles Amin resp. Phenol zur Reaktion gebracht werden und sich die Molverhältnisse innerhalb der in CH-PS 606 169 definierten Grenze halten.

Als Ausgangs- bzw. Grundstoffe für die 1-Oxa-3-aza-tetralin-verbindung dienen Phenol und Phenol-derivate sowie Amine und Formaldehyd.

Beispiele für geeignete Phenole sind:

Einwertige Phenole wie Phenol selbst sowie m- und p-Kresol, m- und p-Ethyl-phenol, m- und p-Isopropylphenol, m- und p-Methoxy-phenol, m- und p-Ethoxy-phenol, m- und p-Isopropyloxy-phenol, m- und p-Chlorphenol und B-Naphtol. Dabei werden die meta-substituierten Phenole vorgezogen, weil bei ihnen keine reaktiven Stellen blockiert sind. Zweiwertige Phenole wie 4,4'-Dihydroxy-diphenylmethan, 3,3'-Dihydroxy-diphenylmethan, 2,2-Bis-(4-hydroxyphenyl)-propan, 4,4'-Dihydroxy-stilben, Hydrochinon, Brenzkatechin, Resorcin.

Niedrig kondensierte Phenol-Formaldehyd-Novolak-Harze, evtl. auch als Mischungen mit Phenol.

Beispiele für besonders geeignete Amine sind:

Anilin, o-, m- und p-Phenylendiamin, Benzidin, 4,4'-Diaminodiphenyl-methan, 2,2-Bis-(aminophenyl)-propan.

Besondere strukturelle Anforderungen werden an die zweite Komponente des chemisch härtbaren Harzes, an das Epoxid-harz gestellt. Damit man Endprodukte mit herausragender Wärmefestigkeit erhält, welche die Qualität bisher bekannter vergleichbarer Harze deutlich übertreffen, sind als zweite Komponente zwei- oder mehrwertige cycloaliphatische Epoxid-harze erforderlich, die mindestens eine anellierte Epoxid-gruppe enthalten, wobei die übrigen Epoxid-gruppen ebenfalls anelliert oder direkt mit einem cycloaliphatischen Rest verknüpft sind:

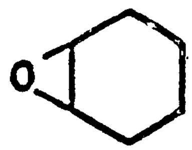

anelliert

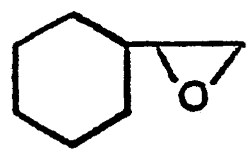

direkt verknüpft

Bevorzugt sind insbesondere Epoxid-harze mit einem Äquivalentgewicht, das zwischen 70 und etwa 250, vorzugsweise 120 und 200 liegt. Die bevorzugten Epoxid-harze sind:

2-(3,4-Epoxy)cyclohexyl-5,5-spiro(3,4-epoxy)cyclohexan-m-dioxan, 1 bis 4fach methyliertes 2-(3,4-Epoxy)cyclohexyl-5,5-spiro(3,4-epoxy)cyclohexan-m-dioxan, 4-(1,2-Epoxyethyl)-1,2-epoxycyclohexan, 1,2:8,9-Diepoxy-p-menthan, 2,2-Bis(3,4-epoxycyclohexyl)propan, Bis-(2,3-epoxycyclopentyl)ether, 1,2:5,6-Diepoxy-4,7-hexahydromethanoindan, Bis-(3,4-epoxy-cyclohexylmethyl)adipat, Bis-(3,4-epoxy-6-methylcyclohexylmethyl)adipat, Bis-(3,4-epoxy-cyclohexylmethyl)-terephth;-lat, Bis-(3,4-epoxy-6-methyl-cyclohexylmethyl)-terephthalat, 3,4-Epoxy-cyclohexancarbon-säure-(3,4-epoxy-cyclohexylmethyl)ester, 3,4-Epoxy-6-methylcyclohexancarbonsäure-(3,4-epoxy-6-methylcyclohexylmethyl)ester, 1,2-Bis-[5(1,2-epoxy)-4,7-hexahydromethanoindanoxy]-ethan, 1,1,1-Tris[[5-(1,2-epoxy)-4,7-hexahydromethanoindanoxy]methyl]-propan und 4,5-Epoxy-hexahydrophthalsäure-bis-(3,4-epoxy-cyclohexylmethyl)ester.

Besonders gute Harze sind solche bei denen das Mengenverhältnis von 1-Oxa-3-aza-tetralin-gruppen enthaltenden Verbindungen und Epoxid-harz ausgedrückt im Molverhältnis von Epoxid- zu 1-Oxa-3-aza-tetralin-gruppe von 0,2 bis 2, vorzugsweise von 0,8 bis 1,5 beträgt. Der Gehalt an 1-Oxa-3-aza-tetralin-gruppen für die Berechnung des Äquivalentverhältnisses wird basierend auf der Menge des zur Reaktion gebrachten primären Amins berechnet; unabhängig davon ob dieses tatsächlich als 1-Oxa-3-aza-tetralin-gruppe eingebaut wird. Dabei wird ferner angenommen, dass pro Mol reagiertem Formaldehyd ein Mol Wasser abgespalten wird. So ergibt sich z. B. in Versuch 6 (siehe Seite 18), das theoretische Äquivalentgewicht der 1-Oxa-3-aza-tetra-lin-verbindung aus folgender Rechnung:

| | | | | |
|---|---|---|---|---|
| 1 | Grammäquivalent | Novolak (Äquivalentgewicht = 100) | = 100 | g |
| + 0,8 | Mol | Anilin | = 74,4 | g |
| + 1,5 | Mol | Formaldehyd | = 45 | g |
| - 1,5 | Mol | Wasser | = -27 | g |
| | | | = 192,4 | g |

N-Äquivalentgewicht = 192,4 : 0,8 = 240,5

Werden bei der Herstellung der 1-Oxa-3-aza-tetralin-verbindungen neben primären Aminen keine anderen Stickstoff-Verbindungen zugesetzt, so kann das Äquivalentgewicht auch aus dem Stickstoffgehalt berechnet werden, der zum Beispiel in bekannter Weise nach Kjeldahl bestimmt wird. Als Verbindungen mit mehr als einer 1-Oxa-3-aza-tetralin-gruppe pro Molekül gelten solche, bei denen das N-Äquivalentgewicht kleiner ist als das mittlere Molekulargewicht.

Besonders bevorzugte Epoxid-harze für die Umsetzung mit den 1-Oxa-3-aza-tetralin-derivaten sind: 2-(3,4-Epoxy)cyclohexyl-5,5-spiro(3,4-epoxy)cyclohexan-m-dioxan, 1 bis 4fach methyliertes 2-(3,4-Epoxy)cyclohexyl-5,5-spiro(3,4-epoxy)cyclohexan-m-dioxan, Bis-(3,4-epoxy-cyclohexylmethyl)adipat, Bis-(3,4-epoxy-6-methylcyclohexylmethyl)adipat, 3,4-Epoxy-cyclohexancarbonsäure-(3,4-epoxy-cyclohexylmethyl)ester und 3,4-Epoxy-6-methylcyclohexancarbonsäure-(3,4-epoxy-6-methylcyclohexylmethyl)ester.

Die am meisten bevorzugten Epoxid-harze können durch die allgemeine Formel

X - Y

definiert werden, worin

X 3,4-Epoxycyclohexyl- oder ein- bis zweifach methyliertes 3,4-Epoxycyclohexyl und

Y einen Rest der Formel

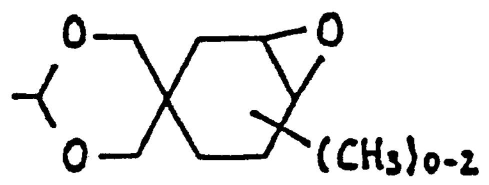

,

Epoxyethyl der Formel

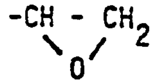

oder eine Gruppe der Formel

$-COO-CH_2-X$ oder $-CH_2-Z-CH_2-X$

darstellt, worin Z den Säurerest einer aliphatischen, cycloaliphatischen oder aromatischen Dicarbonsäure, insbesondere der Adipinsäure, Terephthalsäure oder 4,5-Epoxy-hexahydrophthalsäure, bedeutet.

Die Erfindung betrifft ausserdem die Herstellung eines Harzes, welches dadurch gekennzeichnet ist, dass man Mischungen aus einer mehr als eine 1-Oxa-3-aza-tetralin-gruppe im Molekül enthaltende Verbindung und/oder ein Vorpolymeres davon und einem cycloaliphatischen Epoxid-harz, das mindestens zwei Epoxidgruppen im Molekül enthält, wovon mindestens eine in einem cycloaliphatischen Ring anelliert und die übrigen ebenfalls in einem solchen Ring anelliert oder direkt mit einem solchen verknüpft sind, herstellt und härtet.

Das Verfahren ist weiter dadurch gekennzeichnet, dass man die Komponenten im Mol-Verhältnis von Epoxid zu 1-Oxa-3-aza-tetralin-gruppen von 0,2 bis 2, vorzugsweise von 0,8 bis 1,5 mischt.

Die Härtung wird bei einer Temperatur von 50 bis 300° C, vorzugsweise bei 100 bis 250° C, insbesondere bei 140 bis 230° C durchgeführt.

Für viele Anwendungen ist es vorteilhaft, die Härtung in mindestens 2 Stufen durchzuführen, wobei als Zwischenprodukt ein festes oder höher viskoses aber noch lösliches oder schmelzbares Kunstharz hergestellt wird. Die Vorpolymerisation kann bereits bei der Herstellung der 1-Oxa-3-aza-tetralin-verbindung, vor dem Mischen, beim Mischen oder nach dem Mischen vorgenommen werden. Die Endhärtung wird durch Erhitzen auf etwa 140 bis 230° C durchgeführt.

Die Aushärtung kann durch Zusatz eines die Reaktion beschleunigenden Katalysators verbessert werden. Insbesondere werden damit die Aushärtzeiten verkürzt.

Als Katalysatoren für diesen Zweck kommen beispielsweise in Betracht: Säuren, Friedel-Crafts-Katalysatoren, Amine, Phosphine oder Quartärnisierungsmittel für tertiäre Amine, insbesondere Alkyl-, Aralkyl- und Aryl-Halogenide wie beispielsweise Benzylchlorid, Chlorbenzol, Jodbenzol, Jodoform, Bromoform, Methyljodid oder Methylsulfat.

Bei der Härtung können in üblicher Weise Zusatzstoffe, wie Füllstoffe, Farbstoffe, verstärkende Fasern, Weichmacher und dergleichen zugesetzt werden. Es ist auch möglich, die erfindungsgemässen Produkte mit andern Kunststoffen, Kunstharzen oder zu solchen polymerisierbaren Verbindungen zu kombinieren, insbesondere mit Aldehydkondensationsharzen wie vorzugsweise mit Phenolformaldehyd-harzen und andern Epoxid-harzen sowie deren Härtungsmitteln.

Zusatzstoffe, die mit den erfindungsgemässen Harzen mischbar bzw. in diesen löslich sind, sollen einen Anteil von 50 %, vorzugsweise 10 - 20 % in der Mischung nicht übersteigen.

Die Kunstharze gemäss vorliegender Erfindung sind für vielfältige Zwecke verwendbar beispielsweise als Giess-, Laminier-, Imprägnier-, Beschichtungs-, Klebe-, Lack-, Binde- oder Isolierharz oder als Einbettungs-, Press-, Spritzguss-, Strangguss-, Formsandbinde-, Schaum- und Ablativmasse.

Die neuen Kunstharze sind dort ganz besonders geeignet, wo ausserordentliche Wärmebeständigkeit gefordert wird. So erlaubt die Verwendung von wärmestabileren Isolierharzen etwa bei elektrischen Wicklungen die Anwendung höherer Spannungen oder die Reduktion der Drahtdurchmesser. Neben der Konstruktion von wesentlich wirtschaftlicheren Elektromotoren ermöglichen hochwärmefeste Isolierharze allgemein die Miniaturisierung von elektrischen Vorrichtungen und Konstruktionselementen.

Besonders zweckmässig und vorteilhaft ist auch die Verwendung der neuen hochwärmefesten Kunstharze in mit Glas-, Quarz-, Kohlenstoff- oder Aramid-fasern verstärkten Kunststoffen, weil bei diesen das Stützmaterial

wesentlich wärmestabiler ist als das Kunstharz. Mit der Steigerung der Wärmestabilität solcher Faserverstärkter Kunststoffe können diese für weitere Zwecke als bisher verwendet werden. Sie erlauben noch vermehrten Ersatz von Metallen oder von Keramik-material bei wärmebeanspruchten Konstruktionselementen. Bei diesen Anwendungen ist auch die hohe Biege- und Schlagfestigkeit der erfindungsgemäss erhaltenen Kunststoffharze von besonders grossem Nutzen.

Gegenstand der Erfindung ist daher auch die Verwendung von gehärteten Mischungen aus Verbindungen mit mehr als einer 1-Oxa-3-aza-tetralin-gruppe im Molekül oder deren Vorpolymeren und cycloaliphatischen Epoxid-harzen, wobei das Epoxid-harz mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen verknüpft sind als elektrische Isolierstoffe, Formteile, Schichtpreßstoffe, Leiterplatten, Schaumstoffe, in mit Glas-, Quarz- Kohlenstoff- oder Amid- und Aramid-fasern verstärkten Kunststoffmassen sowie als Klebe-, Beschichtungs- und Dichtungsmasse.

**Beispiele**

1. Herstellung der 1-Oxa-3-aza-tetralin-gruppen enthaltenden Kondensate

**Versuch 1**

In einem mit Rückflusskühler ausgerüsteten Rührgefäss lässt man zu 210 g 30 %-igem Formaldehyd (2,1 Mol) eine Schmelze von 94 g Phenol (1 Mol) und 99 g 4,4'-Diaminodiphenyl-methan (0,5 Mol) innerhalb 15 Minuten bei 80°C zufliessen. Danach lässt man absitzen, trennt die überstehende wässrige, schwach nach Formaldehyd riechende Schicht ab und destilliert das restliche Wasser im Vakuum bei etwa 100°C ab. Die harzartige 1-Oxa-3-aza-tetralin-verbindung hat ein N-Äquivalent von 217.

**Versuch 2**

Unter denselben Bedingungen wie in Versuch 1 lässt man 4,1 Mol Formaldehyd mit 2 Mol Anilin, 1 Mol Phenol und 0,5 Mol Bisphenol A (=2,2-Bis-(4-hydroxyphenyl)-propan) kondensieren. N-Äquivalent = 221

**Versuch 3**

Unter den Bedingungen des Versuchs 1 stellt man das Kondensationsprodukt aus Formaldehyd, Phenol und 1,4-Diaminobenzol im Molverhältnis 2:1:0,5 her. N-Äquivalent = 172.

**Versuch 4**

HERSTELLUNG EINES NOVOLAKS

Die Mischung von 37,6 kg (400 Mol) Phenol, 15 kg (200 Mol) 40-prozentigem Formaldehyd und 2 kg 10-prozentiger Schwefelsäure wird in einem mit Rückflusskühler versehenen Rührgefäss auf 40°C erwärmt. Die Mischung heizt sich durch frei werdende Reaktionswärme bei schwacher Kühlung bis 96°C auf. Sie wird anschliessend noch etwa 30 Min. bei dieser Temperatur gerührt. Nach dem Abkühlen und absitzen lassen wird die wässrige Schicht entfernt. Die untere Schicht besteht aus einem Phenol-Novolak-Gemisch mit einer mittleren Kernzahl von 2 (gemäss der Einwaage berechnet), das 15 % Wasser und 22 % Phenol enthält. Das Phenol-Äquivalent beträgt demnach 117,7.

**Versuch 5**

Zu 157,5 g (2,1 Mol) 40-prozentigem Formaldehyd, der 5 Mol Kaliumhydroxid enthält, lässt man bei 95°C unter Rühren die Mischung von 117,7 g Novolak aus Versuch 4 enthaltend 1 Mol phenolisches Hydroxyl und 93 g (1 Mol) Anilin während 7 Minuten zulaufen und kocht anschliessend 30 Min. am Rückfluss. Dann lässt man absitzen und trennt die wässige Schicht möglichts vollständig ab. Das Harz wird durch Destillation unter Vakuum vom restlichen Wasser befreit, wobei man die Temperatur zuletzt bis auf 123°C steigert. N-Äquivalent = 217.

**Versuch 6**

Unter denselben Bedingungen wie im Versuch 5 stellt man ein im Mittel 1,6 1-Oxa-3-aza-tetralin-gruppen pro Molekül enthaltendes Harz aus 1,5 Mol Formaldehyd, 1 Grammäquivalent Novolak von Versuch 4 und 0,8 Mol Anilin her. N-Äquivalent = 240.

2. Für die vorliegende Erfindung geeignete Epoxid-harze

**Tabelle 1**

| Symbol | Chemische Bezeichnung des Epoxid-harzes |
|---|---|
| A | 4-(1,2-Epoxyethyl)-1,2-epoxycyclohexan |
| B | 1,2:8,9-Diepoxy-p-menthan |
| C | 2,2-Bis-(3,4-epoxycyclohexyl)-propan |
| D | Bis-(2,3-epoxycyclopentyl)ether (flüssige Form) |
| E | Bis-(2,3-epoxycyclopentyl)ether (isomere krist. Form) |
| F | 1,2:5,6-Diepoxy-4,7-hexahydromethanoinddan |
| G | 1,1,1-Trist[[5-(1,2-epoxy)-4,7-hexahydromethanoindanoxyl]-methy]-propan |
| H | 1,2-Bis-[5-(1,2-epoxy)-4,7-hexahydromethanoindanoxyl]-ethan |
| I | 3,4-Epoxycyclohexancarbonsäure-(3,4-epoxycyclohexylmethyl)ester |
| K | 3,4-Epoxy-6-methylcyclohexancarbonsäure-(3,4-epoxy-6-methylcyclohexylmethyl)ester |
| L | 2-(3,4-Epoxy)cyclohexyl-5,5-spiro(3,4-epoxy)-cyclohexan-m-dioxan |
| M | 2-(3,4-Epoxy-4-methylcyclohexyl-5,5-spiro(3,4-epoxy)cyclohexan-m-dioxan |
| N | 2-(3,4-Epoxy)cyclohexyl-5,5-spiro(3,4-epoxy)-4-methylcyclohexan-m-dioxan |
| O | 2-(3,4-Epoxy)-4-methylcyclohexyl-5,5-spiro-(3,4-epoxy)-4-methylcyclohexan-m-dioxan |
| P | 2-(3,4-Epoxy)-4,6-dimethylcyclohexyl-5,5-spiro-(3,4-epoxy)-4-methylcyclohexan-m-dioxan |
| Q | 2-(3,4-Epoxy)-4,6-dimethylcyclohexyl-5,5-spiro-(3,4-epoxy)-4,6-dimethylcyclohexan-m-dioxan |
| R | Bis-(3,4-epoxy-6-methylcyclohexylmethyl)adipat |
| S | Bis-(3,4-epoxycyclohexylmethyl)adipat |
| T | Bis-(3,4-epoxy-cyclohexylmethyl)-terephthalat |
| U | Bis-(3,4-epoxy-6-methylcyclohexylmethyl)-terephthalat |
| V | 4,5-Epoxy-hexahydrophthalsäure-bis-(3,4-epoxy-cyclohexylmethyl)ester |

Strukturformeln der Epoxid-harze F, G, H und L

F

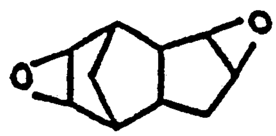

G

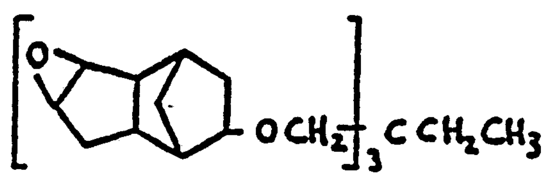

H

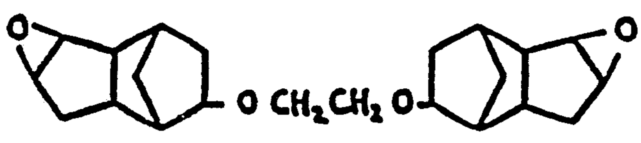

L

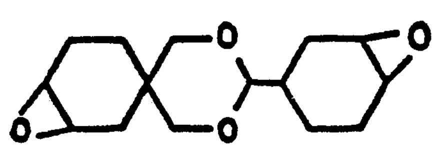

3. Herstellung der Harze

**Beispiel 1**

100 Teile des Kondensats aus Versuch 1 werden bei 140°C mit 100 Teilen 3,4-Epoxycyclohexancarbonsäure-(3,4-epoxycyclohexyl-methyl)ester gemischt und im Vakuum in eine Form für eine 10 mm dicke Platte gegossen. Man härtet die Mischung 1 h bei 180 °C, 1 h bei 200°C und 2 h bei 220°C. Aus der Platte hergestellte Prüflinge zeigen auch bei 250°C keine Zersetzung. Sie haben eine Biegefestigkeit von 115 MPa, einen E-Modul von 4800 MPa, einen elektrischen Durchgangswiderstand von $> 10^{15}$ Ohm und einen Verlustfaktor von $< 10^{-2}$. Die Glasumwandlungstemperatur liegt bei 230°C.

Die Glasumwandlungstemperatur ist definiert als diejenige Temperatur, bei welcher die spezifische Wärme und der thermische Ausdehnungskoeffizient eine starke Zunahme und die mechanischen Eigenschaften einen starken Abfall aufweisen.

**Beispiele 2 bis 17**

Nach der im Beispiel 1 beschriebenen Methode werden Giessharzplatten aus den in Tabelle 2 angegebenen Harzmischungen hergestellt. Die verwendeten Epoxidharze sind in der Tabelle 1 neben weiteren, für den erfindungsgemässen Zweck geeigneten Epoxidharzen aufgeführt. Die Eigenschaften der gehärteten Harze sind aus Tabelle 2 ersichtlich.

**Beispiel 18**

75 g Epoxidharz L (Tabelle 1) werden unter Vakuum bei 120°C mit 100 g der 1-Oxa-3-aza-tetralin-Verbindung gemäss Versuch und 200 g Quarzmehl gemischt und in eine Form gegossen. Der Giessling wird 1 h bei 200°C und 1 h bei 220°C gehärtet.

Er besitzt eine Biegefestigkeit von 60 MPa und zeigt bei 250°C keine Verformung.

**Beispiel 19**

Ein Glasgewebe mit Glycidyl-propyl-silanfinish und einem Flächengewicht von 120 g/m$^2$ wird mit einer 60 %-igen Lösung der Harzmischung gemäss Beispiel 15 imprägniert und im zweistufigen Heissluftkanal bei 100-140°C getrocknet. 8 Lagen des Prepregs werden zwischen Kupferfolien 1 h bei 180°C zu einer Platte verpresst. Das Laminate zeigt im Lötbadtest bei 260°C keine Delaminierung. Der elektrische Durchgangswiderstand ist $> 10^{15}$ Ohm·cm, der Verlustfaktor unter 0,01. Die Biegefestigkeit beträgt 500 MPa.

4. Vergleichsversuche 1 bis 3

Giessharzplatten werden aus Harzgemischen gemäss Tabelle 3 hergestellt, welche nicht der erfindungsgemässen Definition entsprechen. Vergleichsversuche 1 und 2 verwenden andersartige cycloaliphatische Epoxid-harze, Vergleichsversuch 3 ein monofunktionelles 1-Oxa-3-aza-tetralin gemäss CH-Patentschrift 579 113, Beispiel 1. Alle Vergleichsversuchsprodukte zeigen eine deutlich schlechtere Wärmebeständigkeit oder niedrigere Glasumwandlungstemperaturen.

Tabelle 2

TABELLE 2

| Beispiel Nr. | 2 | 3 | 4 | | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| 1-Oxa-3-aza-tetralin gemäss Versuch Nr. | 5 | 5 | 5 | | 5 | 5 | 5 | 5 | 5 | 5 | 5 | 2 | 3 | 6 | 5 | 5 | 5 |
| N-Aequivalent 1) | 217 | 217 | 217 | | 217 | 217 | 217 | 217 | 217 | 217 | 217 | 231 | 172 | 240 | 217 | 217 | 217 |
| Menge g | 100 | 100 | 100 | | 100 | 100 | 100 | 100 | 100 | 100 | 100 | 100 | 100 | 100 | 100 | 100 | 100 |
| Epoxid-Verbindung gemäss Tab. 1 | K | S | L | | L | L | L | L | L | I | O | R | I | L | R + L | K + A | A |
| Epoxid-Aequivalent | 140 3) | 183 3) | 160 2) | | | | | | | 126 3) | 147 3) | 197 3) | 126 3) | 160 3) | - | - | 70 3) |
| Menge g | 100 | 100 | 100 | | 20 | 40 | 66 | 75 | 150 | 100 | 100 | 100 | 100 | 50 | 50+50 | 80+20 | 26 |
| Aequivalentverhältnis EP/N | 1.55 | 1.18 | 1.36 | | 0.27 | 0.54 | 0.9 | 1.0 | 2.0 | 1.7 | 1.5 | 1.2 | 1.36 | 0.75 | - | - | 0.8 |
| Härtungszyklus | 1 h 180°<br>1 h 200°<br>1 h 220° | | → | | | | | | | | | | | | | 24h140°<br>2h200° | 24h140°<br>2h160°<br>2h200° |
| Nachhärtung | | | | 3h250° | | | | | | | | | | | | | |
| Verhalten bei 220°<br>250° | 4)<br>4) | 4)<br>4) | 4)<br>4) | 4)<br>4) | 4)<br>4) | 4)<br>4) | 4)<br>4) | 4)<br>4) | 4)<br>5) | 4)<br>4) | 4)<br>4) | 4)<br>4) | 4)<br>4) | 4)<br>4) | 4)<br>4) | 4)<br>4) | 4)<br>5) |
| Biegefestigkeit MPa 25°<br>220°<br>250° | | 115 | | 100-110<br>65<br>50 | | | | | | 115 | | | | | | | |
| Glasumwandlungstemp. °C | 220° | 190° | 250° | 280° | 210° | 250° | >260° | >260° | >260° | 230° | 220° | 200° | 240° | 180° | 220° | 220° | 170° |

1) aus Herstellung berechnet
2) analytisch bestimmt
3) theoretischer Formel berechnet
4) unverändert
5) Risse und Blasen

1) aus Herstellung berechnet
2) analytisch bestimmt
3) theoretischer Formel berechnet
4) unverändert
5) Risse und Blasen

**Tabelle 3 Vergleichversuche**

| | | | |
|---|---|---|---|
| Nr | 1 | 2 | 3 |
| 1-Oxa-3-aza-tetralin gemäss Versuch Nr | 5 | 5 | N-Phenyl-1-oxa-3-aza-tetralin |
| N-Äquivalent[1]) | | | 211 |
| Menge g | 100 | 100 | 100 |
| Epoxid-Verbindung gemäss Tab. 1 | Tetrahydrophthalsäure-diglysidylester | Hexahydrophthalsäure-diglycidylester | 1 |
| Eposid-Äquivalent | | | 140[3]) |
| Menge g | 100 | 100 | 40 |
| Äquivalentverhältnis EP/N | | | 0.60 |
| Härtungszyklus | 2 h 180°<br>1 h 200° | 2 h 180°<br>1 h 200° | 2 h 160°<br>1 h 200° |
| Verhalten bei 220° | 5) | 5) | 4) |
| Glasumwandlungstemp.° C | | | 110° |

Fussnoten siehe Tab. 2

Die nach den Beispielen der Erfindung erhaltenen Harze weisen im Vergleich zu den strukturell nächstliegenden vorbekannten Harzen, deren Eigenschaften gut bekannt sind, eine im allgemeinen bei weitem höhere Wärmebeständigkeit auf, wobei diese herausragende Eigenschaft parallel läuft mit sehr guten mechanischen Eigenschaften insbesondere mit hoher Biege- und Schlagfestigkeit.

Die Vergleichsversuche bestätigen diesen Sachverhalt.

**Patentansprüche**

1. Chemisch härtbares Harz enthaltend Verbindungen mit mehr als einer 1-Oxa-3-aza-tetralin-gruppe im Molekül und/oder deren Vorpolymere und cycloaliphatische Epoxid-harze, dadurch gekennzeichnet, dass das Epoxid-harz mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen Ring verknüpft sind, wobei das Mengenverhältnis von 1-Oxa-3-aza-tetralin-gruppen enthaltenden Verbindungen und Epoxid-Harz ausgedrückt im Molverhältnis Epoxid- zu 1-Oxa-3-aza-tetralin-gruppen zwischen 0.2 und 2 beträgt.

2. Harz nach Patentanspruch 1, dadurch gekennzeichnet, dass das Äquivalentgewicht des Epoxid-harzes zwischen 70 und etwa 250, vorzugweise 120 und 200 liegt.

3. Harz nach Patentanspruch 1, dadurch gekennzeichnet, dass es als Epoxid-harz 2-(3,4-Epoxy)cyclohexyl-5,5-spiro(3,4-epoxy)-cyclohexan-m-dioxan, 1 bis 4fach methyliertes 2-(3,4-Epoxy)cyclohexyl-5,5-spiro(3,4-epoxy)cyclohexan-m-dioxan, 4-(1,2-Epoxyethyl)-1,2-epoxycyclohexan, 1,2:8,9-Diepoxy-p-menthan, 2,2-Bis-(3,4-epoxycyclohexyl)propan, Bis-(2,3-epoxycyclopentyl)ether, 1,2:5,6-Diepoxy-4,7-hexahydromethanoindan, Bis-(3,4-epoxy-cyclo-hexylmethyl)adipat, Bis(3,4-epoxy-6-methylcyclohexylmethyl)adipat, Bis-(3,4-epoxy-cyclohexylmethyl)-terephthalat, Bis-(3,4-epoxy-6-methyl-cyclohexylmethyl)-terephthalat, 3,4-Epoxy-cyclohexancarbonsäure-(3,4-epoxy-cyclohexylmethyl)ester, 3,4-Epoxy-6-methylcyclohexancarbonsäure-(3,4-epoxy-6-methylcyclohexylmethyl)-ester, 1,2-Bis[5-(1,2-epoxy)-4,7-hexahydromethanoindanoxy]-ethan 1,1,1-Tris[(5-(1,2-epoxy)-4,7-hexahydromethanoindanoxy]methyl]-propan oder 4,5-Epoxy-hexahydrophthalsäure-bis-(3,4-epoxy-cyclo-hexylmethyl)ester enthält.

4. Harz nach Patentanspruch 3, dadurch gekennzeichnet, dass es als Epoxid-harz 2-(3,4-Epoxy)cyclohexyl-5,5-spiro(3,4-epoxy)-cyclohexan-m-dioxan, 1 bis 4fach methyliertes 2-(3,4-Epoxy)cyclohexyl-5,5-spiro(3,4-epoxy)cyclohexan-m-dioxan, Bis-(3,4-epoxy-cyclohexylmethyl)adipat, Bis-(3,4-epoxy-6-methylcyclohexylmethyl)adipat, 3,4-Epoxy-cyclohexancarbonsäure-(3,4-epoxy-cyclohexyl-methyl)ester oder 3,4-Epoxy-6-methylcyclohexancarbonsäure-(3,4-epoxy-6-methylcyclohexylmethyl)ester enthält.

5. Harz nach Patentanspruch 1, dadurch gekennzeichnet, dass es ein Epoxid-harz der allgemeinen Formel

X - Y

enthält, worin

X 3,4-Epoxycyclohexyl- oder ein- bis zweifach methyliertes 3,4-Epoxycyclohexyl und
Y einen Rest der Formel

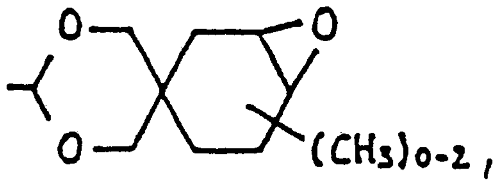

Epoxyethyl der Formel

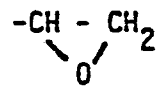

oder eine Gruppe der Formel
$-COO-CH_2-X$ oder $-CH_2-Z-CH_2-X$
darstellt, worin Z den Säurerest einer aliphatischen, cycloaliphatischen oder aromatischen Dicarbonsäure bedeutet.

6. Harz nach Patentansprüchen 1 bis 5, dadurch gekennzeichnet, dass das Mengenverhältnis von 1-Oxa-3-aza-tetralin-gruppen enthaltenden Verbindungen und Epoxid-harz ausgedrückt im Molverhältnis Epoxid- zu 1-Oxa-3-aza-tetralin-gruppen zwischen 0.8 und 1.5 beträgt.

7. Harz nach Patentansprüchen 1 bis 6, dadurch gekennzeichnet, dass die 1-Oxa-3-aza-tetralin-gruppen am Stickstoffatom aromatisch substituiert sind.

8. Verfahren zur Herstellung eines Harzes, dadurch gekennzeichnet, dass man Mischungen aus einer mehr als eine 1-Oxa-3-aza-tetralin-gruppe im Molekül enthaltende Verbindung und/oder ein Vorpolymeres davon und einem cycloaliphatischen Epoxid-harz, das mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen verknüpft sind, herstellt und härtet, wobei das Mischungsverhältnis ausgedrückt im Molverhältnis Epoxid-zu 1-Oxa-3-aza-tetralin-gruppen zwischen 0.2 und 2 beträgt.

9. Verfahren zur Herstellung eines Harzes gemäss Patentanspruch 8, dadurch gekennzeichnet, dass man als Epoxid-harz 2-(3,4-Epoxy)cyclohexyl-5,5-spiro(3,4-epoxy)-cyclohexan-m-dioxan, 1 bis 4fach methyliertes 2-(3,4-Epoxy)cyclohexyl-5,5-spiro(3,4-epoxy)cyclohe-xan-m-dioxan, 4-(1,2-Epoxyethyl)-1,2-epoxycyclohexan, 1,2:8,9-Diepoxy-p-menthan, 2,2-Bis(3,4-epoxycyclohexyl)propan, Bis-(2,3-epoxycyclopentyl)ether, 1,2:5,6-Diepoxy-4,7-hexahydromethanoindan, Bis-(3,4-epoxy-cyclo-hexylmethyl)adipat, Bis-(3,4-epoxy-6-methylcyclohexylmethyl)adipat, Bis-(3,4-epoxy-cyclohexylmethyl)terephthalat, Bis-(3,4-epoxy-6-methyl-cyclohexylmethyl)-terephthalat, 3,4-Epoxy-cyclohexancarbonsäure-(3,4-epoxy-cyclohexyl-methyl)ester, 3,4-Epoxy-6-methylcyclohexancarbonsäure-(3,4-epoxy-6-methylcyclohexylmethyl)ester, 1,2-Bis-(5-(1,2-epoxy)-4,7-hexahydromethanoindanoxy]-ethan, 1,1,1-Tris[[5-(1,2-epoxy)-4,7-hexahydromethanoindanoxy]methyl]-propan oder 4,5-Epoxy-hexahydrophthalsäure-bis-(3,4-epoxy-cyclohexylmethyl)ester verwendet.

10. Verfahren nach Patentanspruch 8, dadurch gekennzeichnet, dass man als Epoxid-harz 2-(3,4-Epoxy)cyclohexyl)-5,5-spiro(3,4-epoxy)-cyclohexan-m-dioxan, 1 bis 4fach methyliertes 2-(3,4-Epoxy)cyclohexyl-5,5-spiro(3,4-epoxy)cyclohexan-m-dioxan, Bis-(3,4-epoxy-cyclohexylmethyl)adipat, Bis-(3,4-epoxy-6-methylcyclohexylmethyl)adipat, 3,4-Epoxy-cyclohexancarbonsäure-(3,4-epoxy-cyclohexylmethyl)ester oder 3,4-Epoxy-6-methylcyclohexancarbonsäure-(3,4-epoxy-6-methylcyclohexylmethyl)ester verwendet.

11. Verfahren nach Patentanspruch 8, dadurch gekennzeichnet, dass man ein Epoxid der allgemeinen Formel
X - Y
verwendet, worin
X 3,4-Epoxycyclohexyl- oder ein- bis zweifach methyliertes 3,4-Epoxycyclohexyl und
Y einen Rest der Formel

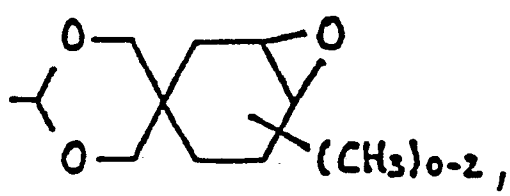

Epoxyethyl der Formel

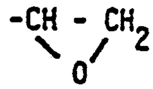

oder eine Gruppe der Formel

$-COO-CH_2-X$ oder $CH_2-Z-CH_2-X$

darstellt, worin Z den Säurerest einer aliphatischen, cycloaliphatischen oder aromatischen Dicarbonsäure bedeutet.

12. Verfahren nach Patentanspruch 8, dadurch gekennzeichnet, dass man die Komponenten im Mol-Verhältnis von Epoxid- zu 1-Oxa-3-aza-tetralin-gruppen von 0.8 bis 1.5 mischt.

13. Verfahren nach Patentansprüchen 8 bis 12, dadurch gekennzeichnet, dass die 1-Oxa-3-aza-tetralin-gruppen am Stickstoffatom substituiert sind.

14. Verfahren nach Patentanspruch 8, dadurch gekennzeichnet, dass man die Härtung bei erhöhter Temperatur durchführt.

15. Verfahren nach Patentanspruch 14, dadurch gekennzeichnet, dass man die Härtung bei Temperaturen von 50 bis 300°C, vorzugsweise im Temperaturbereich von 100 bis 250°C, insbesondere bei 140 bis 230°C durchführt.

16. Verfahren nach Patentanspruch 10, dadurch gekennzeichnet, dass man die Härtung in Gegenwart eines die Reaktion beschleunigenden Katalyten durchführt.

17. Verwendung von Mischungen aus Verbindungen mit mehr als einer 1-Oxa-3-aza-tetralin-gruppe im Molekül und/oder deren Vorpolymeren und cycloaliphatischen Epoxid-harzen, wobei das Epoxid-harz mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen verknüpft sind als Giess-, Laminier-, Imprägnier-, Beschichtungs-, Klebe-, Lack-, Binde- oder Isolierharz oder als Einbettungs-, Press-, Spritzguss-, Strangguss-, Formsandbinde-, Schaum- und Ablativmasse.

18. Verwendung von gehärteten Mischungen aus Verbindungen mit mehr als einer 1-Oxa-3-aza-tetralin-gruppe im Molekül und/oder deren Vorpolymeren und cycloaliphatischen Epoxid-harzen, wobei das Epoxid-harz mindestens zwei Epoxid-gruppen im Molekül enthält, wovon mindestens eine in einem cycloaliphatischen Ring anelliert ist und die übrigen ebenfalls in einem solchen Ring anelliert oder direkt mit einem solchen verknüpft sind, als elektrische Isolierstoffe, Formteile, Schichtpreßstoffe, Leiterplatten, in mit Glas-, Quarz-, Kohlenstoff-, Amid- und Aramidfaser verstärkten Kunststoffmassen, sowie als Klebe-, Beschichtungsund Dichtungsmasse.

**Claims**

1. A chemically curable resin containing compounds with more than one 1-oxa-3-aza-tetraline group in the molecule and/or the prepolymers thereof as well as cycloaliphatic epoxy resins, characterized in that the epoxy resin contains at least two epoxy groups in the molecule, at least one of it being anellated in a cycloaliphatic ring and the remaining being also anellated in such a ring or directly attached to such a ring, and whereby the quantitavive ratio of 1-oxa-3-aza-tetraline group containing compounds and epoxy resin, expressed by the mole ratio of epoxy groups to 1-oxa-3-aza-tetraline groups, is between 0.2 and 2.

2. Resin according to patent claim 1, characterized in that the equivalent weight of the epoxy resin is between 70 and about 250, preferably between 120 and 200.

3. Resin according to patent claim 1, characterized in that it contain as epoxy resin 2-(3,4-epoxy)cyclohexyl-5,5-spiro(3,4-epoxy)cyclohexane-m-dioxane, 1 to 4 times methylated 2-(3,4-epoxy)cyclohexyl-5,5-spiro(3,4-epoxy) cyclohexane-m-dioxane, 4-(1,2-epoxyethyl)-1,2-epoxycyclohexane, 1,2,8,9-diepoxy-p-methane, 2,2-bis-(3,4-epoxycyclohexyl)propane, bis-(2,3-epoxycyclopentyl) ether, 1,2,5,6-diepoxy-4,7-hexahydromethanoindane, bis-(3,4-epoxy-cyclohexylmethyl) adipate, bis-(3,4-epoxy-6-methylcyclohexylmethyl) adipate, bis-(3,4-epoxy-cyclohexylmethyl) terephthalate, bis-(3,4-epoxy-6-methyl-cyclohexyl-methyl)-terephthalate, 3,4-epoxy-cyclohexane carboxylic acid-(3,4-epoxy-cyclohexylnethyl) ester, 3,4-epoxy-6-methylcyclohexane carboxylic acid-(3,4-epoxy-6-methylcyclohexylmethyl) ester, 1,2-bis-(5-(1,2-epoxy)-4,7-hexahydromethanoindaneoxy)-ethane, 1,1,1-tris((5-(1,2-epoxy)-4,7-hexahydromethanoindane oxy)methyl)-propane or 4,5-epoxy-hexahydrophtalic acid-bis-(3,4-epoxy-cyclohexylmethyl) ester.

4. Resin according to patent claim 3, characterized in that it contains as epoxy resin 2-(3,4-epoxy)cyclohexyl-5,5-spiro(3,4-epoxy) cyclohexane-m-dioxane, 1 to 4 times methylated 2-(3,4-epoxy) cyclohexyl-5,5-spiro (3,4-epoxy)cyclohexane-m-dioxane, bis-(3,4-epoxy-cyclohexylmethyl)adipate, bis-(3,4-epoxy-6-methyl-cyclohexylmethyl)adipate, 3,4-epoxy-cyclohexane-carboxylic acid-(3,4-epoxy-cyclohexylmethyl) ester or 3,4-epoxy-6-methyl-cyclohexane carboxylic acid-(3,4-epoxy-6-methylcyclohexyl-methyl) ester.

5. Resin according to patent claim 1, characterized in that it contains an epoxy resin of the general formula

X - Y

wherein:

X is a 3,4-epoxycyclohexyl group or a 3,4-epoxycyclohexyl group substituted by mono- or di-methyl; and

Y is a group represented by the formula

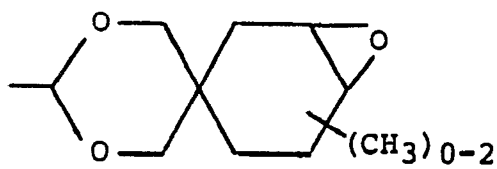

or a epoxyethyl represented by the formula

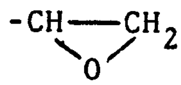

or a group represented by the formulae
$-COO-CH_2-X$ or $-CH_2-Z-CH_2-X$ wherein:
Z is the acid residue of an aliphatic, cycloaliphatic or aromatic dicarboxylic acid.

6. Resin according to patent claims 1 to 5, characterized in that the quantitavive ratio of 1-oxa-3-aza-tetraline group containing compounds and epoxy resin, expressed by the mole ratio of epoxy groups to 1-oxa-3-aza-tetraline groups, is between 0.8 and 1.5.

7. Resin according to patent claims 1 to 6, characterized in that the 1-oxa-3-aza-tetraline groups are aromatically substituted on the nitrogen atom.

8. A method for the preparation of a resin, characterized in that one prepares and cures mixtures of a compound with more than one 1-oxa-3-aza-tetraline group in the molecule and/or the prepolymers thereof and cycloaliphatic epoxy resins containing at least two epoxy groups in the molecule, at least one of it being anellated in a cycloaliphatic ring and the remaining being also anellated in such a ring or directly attached to such a ring, whereby the mixture ratio is between 0.2 and 2.

9. Method according to claim 8, characterized in that on uses as epoxy resin 2-(3,4-epoxy)cyclohexyl-5,5-spiro(3,4-epoxy)-cyclohexane-m-dioxane, 1 to 4 times methylated 2-(3,4-epoxy)-cyclohexyl-5,5-spiro(3,4-epoxy) cyclohexane-m-dioxane, 4-(1,2-epoxyethyl)-1,2-epoxycyclohexane, 1,2,8,9-diepoxy-p-methane, 2,2-bis-(3,4-epoxycyclohexyl)propane, bis-(2,3-epoxycyclopentyl) ether, 1,2,5,6-diepoxy-4,7-hexahydromethanoindane, bis-(3,4-epoxy-cyclohexylmethyl) adipate, bis-(3,4-epoxy-6-methylcyclo-hexylmethyl) adipate, bis-(3,4-epoxy-cyclohexylmethyl) terephthalate, bis-(3,4-epoxy-6-methyl-cyclohexylmethyl)-terephthalate, 3,4-epoxy-cyclohexane carboxylic acid-(3,4-epoxy-cyclohexylmethyl) ester, 3,4-epoxy-6-methylcyclohexane carboxylic acid-(3,4-epoxy-6-methylcyclohexylmethyl) ester, 1,2-bis-(5(1,2-epoxy)-4,7-hexahydromethanoindane oxy)-ethane, 1,1,1-tris((5-(1,2-epoxy)-4,7-hexahydromethanoindane oxy)methyl)-propane or 4,5-epoxy-hexahydrophtalic acid-bis-(3,4-epoxy-cyclohexylmethyl) ester.

10. Method according to claim 8, characterized in that on uses as epoxy resin 2-(3,4-epoxy)cyclohexyl-5,5-spiro(3,4-epoxy) cyclohexane-m-dioxane, 1 to 4 times methylated 2-(3,4-epoxy) cyclohexyl-5,5-spiro (3,4-epoxy)cyclohexane-m-dioxane, bis-(3,4-epoxy-cyclohexylmethyl)adipate, bis-(3,4-epoxy-6-methyl-cyclohexylmethyl)adipate, 3,4-epoxy-cyclohexane-carboxylic acid-(3,4-epoxy-cyclohexylmethyl) ester or 3,4-epoxy-6-methyl-cyclohexane carboxylic acid-(3,4-epoxy-6-methylcyclohexyl-methyl) ester.

11. A method according to patent claim 8, characterized in that one uses an epoxy resin of the general formula
X - Y
wherein:
X is a 3,4-epoxycyclohexyl group or a 3,4-epoxycyclohexyl group substituted by mono- or di-methyl; and
Y is a group represented by the formula

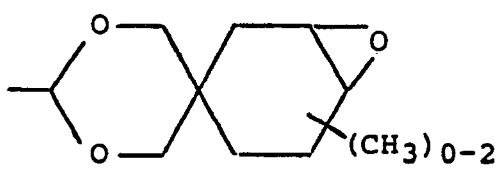

or an epoxyethyl represented by the formula

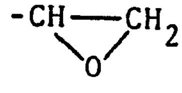

or a group represented by the formulae
$-COO-CH_2-X$ or $-CH_2-Z-CH_2-X$ wherein:

Z is the acid residue of an aliphatic, cycloaliphatic or aromatic dicarboxylic acid.

12. Method according to patent claim 8, characterized in that one mixes the components in the mole ratio of epoxy groups to 1-oza-3-aza-tetraline groups of 0.8 to 1.5.

13. Method according to patent claims 8 to 12, characterized in that the 1-oxa-3-aza-tetraline groups are aromatically substituted on the nitrogen atom.

14. Method according to patent claim 8, characterized in that the curing is effected at elevated temperature.

15. Method according to patent claim 14, characterized in that the curing is effected at temperatures of from 50 to 300° C, preferably in the temperature range of from 100 to 250° C, particularly at 140 to 230° C.

16. Method according to patent claim 10, characterized in that the curing is effected in the presence of a catalyst which accelerates the reaction.

17. Use of mixtures of compounds with more than one 1-oxa-3-aza-tetraline group in the molecule and/or the prepolymers thereof and cycloaliphatic epoxy resins containing at least two epoxy groups in the molecule, at least one of it being anellated in a cycloaliphatic ring and the remaining being also anellated in such a ring or directly attached to such a ring, as casting, laminating, impregnating, coating, gluing, painting, binding or insulating masses or as embedding, pressing, injection molding, extruding, sand mold binding, foam and ablative masses.

18. Use of mixtures of compounds with more than one 1-oxa-3-aza-tetraline group in the molecule and/or the prepolymers thereof and cycloaliphatic epoxy resins containing at least two epoxy groups in the molecule, at least one of it being anellated in a cycloaliphatic ring and the remaining being also anellated in such a ring or directly attached to such a ring, as electric insulating material, shaped articles, compressed multi-layer materials, circuit boards, in plastic masses reinforced by glass, quartz, carbon, amide and aramide fibers, as well as bonding, coating and sealing mass.

**Revendications**

1. Résine durcissable chimiquement contenant des composés dont la molécule contient plus d'un groupe oxa-1 aza-3 tétraline et/ou des prépolymères de ces composés, et des résines époxydes cycloaliphatiques, caractérisée en ce que la molécule de la résine époxyde contient au moins deux groupes époxy dont au moins un est en pont sur un cycle cycloaliphatique et les autres sont également en pont sur un tel cycle ou liés directement à ce cycle, le rapport de la quantité de composés contenant des groupes oxa-1 aza-3 tétraline à la quantité de résine époxyde, exprimé par le rapport molaire groupes époxy/groupes oxa-1 aza-3 tétraline, étant compris entre 0,2 et 2,0.

2. Résine selon la revendication 1, caractérisée en ce que la masse équivalente de la résine époxyde est comprise entre 70 et environ 250, de préférence entre 120 et 200.

3. Résine selon la revendication 1, caractérisée en ce qu'elle contient comme résine époxyde une des résines suivantes: 2-(3,4-époxy)cyclohexyl-5,5-spiro(3,4-époxy)-cyclohexan-m-dioxane, 2-(3,4-époxy)cyclohexyl-5,5-sairo(3,4-époxy)-cyclohexan-m-dioxane monoéthylé à tétraéthylé, 4-(1,2-époxyéthyl)-1,2-époxycyclohexane, 1,2:8,9-diépoxy-p-menthane, 2,2-bis-(3,4-époxycyclohexyl)propane, bis-(2,3-époxycyclopentyl)éther, 1,2:5,6-diépoxy-4,7-hexahydrométhanoindène, adipate de bis-(3,4-époxy-cyclohexylméthyle), adipate de bis-(3,4-époxy-6-méthylcyclohexylméthyle), téréphtalate de bis-(3,4-époxy-cyclohexylméthyle), téréphtalate de bis-(3,4-époxy-6-méthyl-cyclohexylméthyle), 3,4-époxy-cyclohexancarboxylate-(3,4-époxy-cyclohexylméthyl)ester, 3,4-époxy-6-méthyl-cyclohexancarboxylate-(3,4-époxy-6-méthylcyclohexyl-méthyl)ester, 1,2-bis[5-(1,2-époxy)-4,7-hexahydrométhanoindènoxy)-éthane, 1,1,1-tris[[5-(1,2-époxy)-4,7-hexahydrométhanoindènoxy]méthyl]-propane ou 4,5-époxy-hexahydrophtalate-bis-(3,4-époxy-cyclohexylméthyl)ester.

4. Résine selon la revendication 3, caractérisée en ce qu'elle contient comme résine époxyde une des résines suivantes: 2-(3,4-époxy)cyclohexyl-5,5-spiro(3,4-époxy)-cyclohexan-m-dioxane, 2-(3,4-époxy)cyclohexyl-5,5-spiro(3,4-époxy)-cyclohexan-m-dioxane méthylé à tétraméthylé, adipate de bis-(3,4-époxy-cyclohexylméthyle), adipate de bis-(3,4-époxy-6-méthylcyclohexylméthyle), 3,4-époxy-cyclohexancarboxylate-(3,4-époxy-cyclohexylméthyl)ester, ou 3,4-époxy-6-méthylcyclohexancarboxylate-(3,4-époxy-6-méthyl-cyclohexylméthyl)ester.

5. Résine selon la révendication 1, caractérisée en ce qu'elle contient une résine époxyde de formule générale

X - Y,

dans laquelle

X est un 3,4-époxycyclohexyle ou un 3,4-époxycyclohexyle monométhylé ou diméthylé et

Y un radical de formule

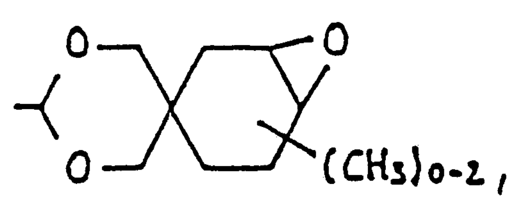

un époxyéthyle de formule

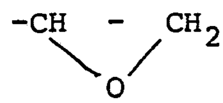

ou un groupé dé formule
-COO-$CH_2$-X ou -$CH_2$-Z-$CH_2$-X,
Z étant le radical acide d'un acide dicarboxylique aliphatique, cycloaliphatique ou aromatique.

6. Résine selon les revendications 1 à 5, caractérisée en ce que le rapport de la quantité de composés contenant des groupes oxa-1 aza-3 tétraline à la quantité de résine époxyde, éxprime par le rapport molaire groupes époxy/groupes oxa-1 aza-3 tétraline, est compris entre 0,8 et 1,5.

7. Résine selon les revendications 1 à 6, caractérisée en ce que les groupes oxa-1 aza-3 tétraline sont substitués aromatiquement sur l'atome d'azote.

8. Procédé de préparation d'une résine, caractérisé en ce qu'on prépare et durcit des mélanges d'un composé dont la molécule contient plus d'un groupe oxa-1 aza-3 tétraline et/ou d'un prépolymère de ce composé, et d'une résine époxyde cycloaliphatique dont la molécule contient au moins deux groupes époxy dont au moins un est en pont sur un cycle cycloaliphatique et les autres sont également en pont sur un tel cycle ou liés directement a ce cycle, le rapport molaire groupes époxy/groupes oxa-1 aza-3 tétraline dans le mélange étant compris entre 0,2 et 2,0.

9. Procédé de préparation d'une résine selon la revendication 8, caractérisé en ce qu'on utilise comme résine époxyde une des résines suivantes: 2-(3,4-époxy)cyclohexyl-5,5-spiro(3,4-époxy)-cyclohexan-m-dioxane, 2-(3,4-époxy)cyclohexyl-5,5-spiro(3,4-époxy)cyclohexan-m-dioxane monoéthylé ou tétraméthylé, 4-(1,2-époxyéthyl)-1,2-époxycyclohexane, 1,2:8,9-diépoxy-p-menthane, 2,2-bis(3,4-époxycyclohexyl)propane, bis-(2, 3-époxycyclopentyl)éther, 1,2:5,6-diépoxy-4,7-hexahydrométhanoindéne, adipate de bis-(3,4-époxy-cyclohexylméthyle), adipate de bis-(3,4-époxy-6-méthylcyclohexylméthyle), téréphtalaté de bis-(3,4-époxy-cyclohexylméthyle), téréphtalate de bis-(3,4-époxy-6-méthyl-cyclohexylméthyle), 3,4-époxy-cyclohexancarboxylate-(3,4-époxy-cyclohexylméthyl)ester, 3,4-époxy-6-méthyloyclohexancarboxylate-(3,4-époxy-6-méthylcyclohexylméthyl)ester, 1,2-bis[5-(1,2-époxy)-4,7-hexahydrométhanoindènoxy]-éthane, 1,1,1-tris[[5-(1,2-époxy)-4,7-hexahydrométhanoindènoxy]méthyl]-propane ou 4,5-époxy-hexahydrophtalate-bis-(3,4-époxycyclohexylméthyl)ester.

10. Procédé selon la revendication 8, caractérisé en ce qu'on utilise comme résine époxyde une des résines suivantes: 2-(3,4-époxy)cyclohexyl)-5,5-spiro(3,4-époxy)-cyclohexan-m-dioxane, 2-(3,4-époxy)cyclohexyl-5,5-spiro-(3,4-époxy)cyclohexan-m-dioxane monoéthylé à tétraméthyle, adipate de bis(3,4-époxy-cyclohexylméthyle), adipate de bis-(3,4-époxy-6-méthylcyclohexylméthyle), 3,4-époxy-cyclohexancarboxylate-(3,4-époxycyclohexylméthyl)ester ou 3,4-époxy-6-méthylcyclohexancarboxylate-(3,4-époxy-6-méthylcyclohexylméthyl)ester.

11. Procédé selon la revendication 8, caractérisé en ce qu'on utilise une résine époxyde de formule générale
X - Y,
dans laquelle
X est un 3,4-époxycyclohexyle ou un 3,4-époxycyclohexyle monométhylé ou diméthydé, et
Y un radical de formule

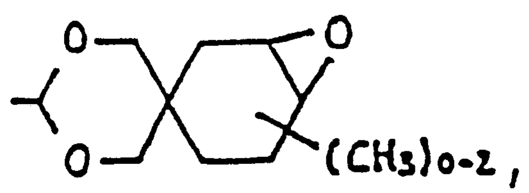

un époxyéthyle de formule

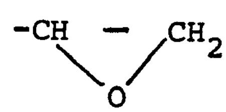

ou un groupe de formule
-COO-$CH_2$-X ou -$CH_2$-Z-$CH_2$-X,
Z étant le radical acide d'un acide dicarboxylique aliphatique, cycloaliphatique ou aromatique.

12. Procédé selon la revendication 8, caractérisé en ce qu'on mélange les constituants dans un rapport molaire groupes époxy/groupes oxa-1 aza-3 tétraline de 0,8 à 1,5.

13. Procédé selon les revendications 8 à 12, caractérisé en ce que les groupes oxa-1 aza-3 tétraline sont substitués sur l'atome d'azote.

14. Procédé selon la revendication 8, caractérisé en ce qu'on effectue le durcissement à température élévée.

15. Procédé selon la revendication 14, caractérisé en ce qu'on effectue le durcissement à des températures de 50 a 300° C, de préférence de 100 a 250° C, en particulier de 140 à 230° C.

16. Procédé selon la revendication 10, caractérisé en ce qu'on fait le durcissement en présence d'un catalyseur accélérant la réaction.

17. Utilisation de mélanges de composés dont la molécule contient plus d'un groupe oxa-1 aza-3 tétraline et/ou de prépolymères de ces composés, et de résines époxydes cycloaliphatiques, la molécule de la résine époxyde contenant au moins deux groupes époxy dont au moins un est en pont sur un cycle cycloaliphatique et les autres sont également en pont sur un tel cycle ou liés directement à ce cycle, comme résine de coulée, résine pour stratifiés, résine d'imprégnation, résine de revêtement, résine adhésive, résine pour peintures et vernis, résine liant ou résine isolante ou comme matière d'énrobage, matière à mouler, matière à mouler par injection, matière à extruder, liant pour sable de moulage, matière alvéolaire ou matière ablative.

18. Utilisation de mélanges durcis de composés dont la molécule contient plus d'un groupe oxa-1 aza-3 tétraline et/ou de prépolymères de ces composés, et de résines époxydes cycloaliphatiques, la molécule de la résine époxyde contenant au moins deux groupes époxy dont au moins un est en pont sur un cycle cycloaliphatique et les autres sont également en pont sur un tel cycle ou liés directement à ce cycle, comme isolants électriques, pièces moulées, stratifiés, plaquettes à circuit imprimé, dans des matières plastiques renforcées par des fibres de verre, de quartz, de carbone, d'amides ou d'aramides, ainsi que comme matière adhésive, matière de revêtement ou matière d'étanchéité.